# EUROPEAN PATENT APPLICATION

(11) **EP 3 995 509 A1**
(43) Date of publication of application: **11.05.2022**
(21) Application number: 20837110.4
(22) Date of filing: 26.06.2020
(51) Int. Cl.: C07K 14/705, C07K 16/00, C07K 17/06, C07K 19/00, C12M 1/00, C12M 3/00, C12N 5/0793

(54) **METHOD FOR INDUCING NEURONAL SYNAPSE FORMATION AND MICROBEADS USED IN SAID METHOD**

(30) Priority: 05.07.2019 JP 2019126416
(71) Applicant: Jiksak Bioengineering, Inc., Kawasaki-shi, Kanagawa, 212-0032 (JP)
(72) Inventor: YUMOTO Norihiro, Kawasaki-shi, Kanagawa 212-0032 (JP); KAWADA Jiro, Kawasaki-shi, Kanagawa 212-0032 (JP)
(74) Representative: Parchmann, Stefanie
(86) International application number: PCT/JP2020/025313
(87) International publication number: WO 2021/006075

(57) **Abstract**

An object of the present invention is to provide a method for inducing the formation of the presynaptic apparatus in human neurons.

According to the present invention, a method for inducing the formation of the presynaptic apparatus in human neurons, comprising co-culturing the peripheral neurons with microbeads in which at least one LRRTM molecule selected from the group consisting of the LRRTM (Leucine-rich repeat transmembrane neuronal protein) family molecules or a fusion protein containing the same is fixed to the surface (wherein, the LRRTM molecule or the fusion protein containing the same molecule is fixed to the surface of the microbead via a linker), and a microbead used for the method, are provided.

## Description

### [Technical Field]

The present invention relates to a method for inducing the formation of the presynaptic apparatus in a neuron. The present invention also relates to a microbead used for this method.

### [Background Art]

Neurons in central as well as peripheral nervous system regulate human emotions and behaviors by mutual exchange of signals via synapses. The synapse is a gap formed between an axon as the presynaptic apparatus and a dendrite as the post-synaptic apparatus (central nervous system) or a target cell of skeletal muscles and organs (peripheral nervous system), and a signal is transmitted by the bond of a chemical substance released from the presynaptic apparatus to a receptor present on the postsynaptic apparatus. For synapse formation, conversion into intracellular signals is triggered by the interaction of specific membrane proteins expressed on the presynapse and the postsynapse, subsequently, proteins involved in the release of chemical substances accumulate on the anterior region and proteins that receive them and transmit them downstream accumulate on the posterior region, both of which form a special structure, resulting in completing the synapse formation.

There are multiple membrane ligands and their receptors associated with the initiation of synapse formation in the central nervous system, and acting cell species and synaptic species (excitatory synapses or inhibitory synapses) to be formed vary depending on the combination of them (Non-Patent Document 1), hence, the mechanisms of temporal and spatial regulation of specific synapse formation in specific cells remain unclear in many points. Furthermore, many of the findings on synapse formation so far mainly use primary cultures of rodent hippocampal neurons and cerebral cortical neurons (Non-Patent Documents 2 and 3), and it is unclear whether those mechanisms are common to humans.

Regarding peripheral nervous system synapse formation, it has been reported that a membrane protein Lrp4 (LDL-receptor related protein 4) expressing in the skeletal muscle which is the postsynaptic-region induces synapse formation via an unknown receptor expressing in the motor neuron, only in mice (Non-Patent Document 4), but most of it is unclear.

The LRRTM family is one of the synaptic organizer molecular families on the postsynaptic terminal side of the central nervous system, and four types of LRRTM 1 to LRRTM4 have been reported for humans and mice. LRRTM2 (Leucine-rich repeat transmembrane protein 2) is a membrane protein expressing in the postsynaptic-region and has been reported to induce the formation of the excitatory presynaptic apparatus in the hippocampal primary cultured nerves of mice, which are central neurons (Non-Patent Document 5). However, it is unclear whether LRRTM2 also induces the formation of the presynaptic apparatus in human central neurons. In addition, it has been reported that, like neuroligin 1, LRRTM2 induces the excitatory synapse using mouse hippocampal neurons (Non-Patent Document 6), in which it has been reported that, like neuroligin 1, the LRRTM2 ligand is a neurexin.

So far, there have been no reports on factors that induce synapse formation in human neurons, in addition, there have been no reports on methods that induce the formation of the presynaptic apparatus. In order to elucidate the formation and maintenance of human synapses, it is desired to establish methods for identifying the factors involved in them or methods for inducing them.

### [Citation List]

### Non-patent Document

Non-Patent Document 1: Sudhof TC, Towards an Understanding of Synapse Formation. Neuron. 2018 Oct 24; 100(2):276-293
Non-Patent Document 2: Uemura et al., Trans-synaptic interaction of GluRdelta2 and Neurexin through Cbln1 mediates synapse formation in the cerebellum. Cell. 2010 Jun 11;141(6): 1068-79.
Non-Patent Document 3: Siddiqui et al., An LRRTM4-HSPG complex mediates excitatory synapse development on dentate gyrus granule cells. Neuron. 2013 Aug 21;79(4):680-95.
Non-Patent Document 4: Yumotoet al., Lrp4 is a retrograde signal for presynaptic differentiation at neuromuscular synapses. Nature. 2012 Sep 20;489(7416):438-42.
Non-Patent Document 5: Linhoff et al., An unbiased expression screen for synaptogenic proteins identifies the LRRTM protein family as synaptic organizers. Neuron. 2009 Mar 12;61(5):734-49.
Non-Patent Document 6: J. Koet al., LRRTM2 Function as a Neurexin Ligandd in Promoting Excitatory Synapse Formation, Neuron 64, 791-798 (2009)

### [Summary of the Invention]

### [Technical Problem]

An object of the present invention is to identify a factor that induces the formation of the presynaptic apparatus in human neurons and to provide a method for inducing the formation of the presynaptic apparatus in human neurons using it.

### [Solution to Problem]

The present inventors have intensively studied, and resultantly found that LRRTM2 (Leucine Rich Repeat Transmembrane Protein 2), which is one of the LRRTM family molecules, induces the formation of the presynaptic apparatus in human neurons. Then, by co-culturing with a neuron using a microbead on which LRRTM2 has been fixed via a specific linker, induction of the formation of the presynaptic apparatus of a neuron has been succeeded, leading to completion of the present invention. The present invention includes the following embodiments.

(1) A method for inducing the formation of the presynaptic apparatus in mammalian neurons (preferably, human neurons), comprising co-culturing the neurons with microbeads in which at least one LRRTM molecule selected from the group consisting of LRRTM (Leucine-rich repeat transmembrane neuronal protein) family molecules or a fusion protein containing the same molecule is fixed to the surface thereof (wherein, the LRRTM molecule or the fusion protein containing the same molecule is fixed to the surface of the microbead via a linker).
(2) The method according to (1), wherein the fusion protein containing the LRRTM molecule is a fusion protein containing the LRRTM molecule and an Fc region of IgG (preferably, human IgG) and the linker is an anti-IgG Fc antibody (preferably, an anti-human IgG Fc antibody) fixed to the surface of the microbead, and the fusion protein containing the LRRTM molecule is fixed to the surface of the microbead via a bond between the Fc region of IgG (preferably, human IgG) and the anti-IgG Fc antibody (preferably, the anti-human IgG Fc antibody).
(3) The method according to (1), wherein the linker is a macromolecule (for example, protein, optionally modified polyethylene glycol, optionally modified sugar chain, optionally modified nucleic acid, etc.), and the length of the linker is 10 nm or more.
(4) The method according to (1), wherein the distance between the LRRTM molecule or the LRRTM molecule which is a part of the fusion protein fixed to the surface of the microbead, and the surface of the microbead, is 10 nm or more.
(5) The method according to any one of (1) to (4), wherein the neurons are human peripheral neurons.
(6) The method according to any one of (1) to (4), wherein the neurons are human central neurons.
(7) The method according to (5), wherein the neurons are human motor neurons.
(8) The method according to (6), wherein the neurons are human glutamatergic neurons.
(9) The method according to any one of (1) to (8), wherein the LRRTM molecule is an LRRTM 1 molecule, an LRRTM2 molecule, an LRRTM3 molecule, or an LRRTM4 molecule.
(10) The method according to (9), wherein the LRRTM molecule is LRRTM2.
(11) The method according to any one of (1) to (10), wherein the neurons are neurons differentiated from a human-derived pluripotent stem cell.
(12) The method according to (11), wherein the neurons are neurons differentiated from a human iPS cell or a human ES cell.
(13) The method according to any one of (1) to (12), wherein the fusion protein is a fusion protein obtained by expressing, in a host, a plasmid containing a DNA including a DNA which codes the amino acid sequence of the LRRTM molecule and a DNA which codes the amino acid sequence of the Fc region of human IgG.
(14) The method according to (13), wherein the LRRTM molecule is LRRTM2.
(15) The method according to any one of (1) to (14), further comprising a step of immunostaining the presynaptic apparatus of the cells after culturing with an anti-synapsin antibody to confirm the induction of the formation of the presynaptic apparatus.
(16) The method according to any one of (1) to (14), further comprising a step for confirming that the induced synapse containing the presynaptic apparatus has a functionality, after culturing, by any of the following detections of:
   (a) detecting a neurotransmitter released from the synapse,
   (b) detecting the expression of a protein associated with the release of a neurotransmitter from a synapse, or
   (c) adding a labeling agent (for example, a dye molecule) that labels synaptic vesicles to a medium, and visualizing and detecting the synaptic vesicles that are taken up after the release of a neurotransmitter from the synapse.
(17) A microbead in which at least one LRRTM molecule selected from the group consisting of the LRRTM (Leucine-rich repeat transmembrane neuronal protein 2) family molecules or a fusion protein containing the same molecule is fixed to the surface thereof via a linker, the microbead being used for culturing mammalian neurons (preferably, human neurons) to induce synapse formation.
(18) The microbead according to(17), wherein the fusion protein is a fusion protein containing the LRRTM molecule and an Fc region of IgG (preferably, human IgG) and the linker is an anti-IgG Fc antibody (preferably, an anti-human IgG Fc antibody) fixed to the surface of the microbead, and the fusion protein containing the LRRTM molecule is fixed to the surface of the microbead via a bond between the Fc region of IgG (preferably, human IgG) and the anti-IgG Fc antibody (the anti-human IgG Fc antibody).
(19) The microbead according to (17), wherein the linker is a macromolecule (for example, protein, optionally modified polyethylene glycol, optionally modified sugar chain, optionally modified nucleic acid, etc.), and the length of the linker is 10 nm or more.
(20) The microbead according to (17), wherein the distance between the LRRTM molecule or the LRRTM molecule which is a part of the fusion protein fixed to the surface of the microbead, and the surface of the microbead, is 10 nm or more.
(21) The microbead according to any one of (17) to (20), wherein the neurons are human peripheral neurons.
(22) The microbead according to any one of (17) to (20), wherein the neurons are human central neurons.
(23) The microbead according to (21), wherein the neurons are human motor neurons.
(24) The microbead according to (22), wherein the neurons are human glutamatergic neurons.
(25) The microbead according to any one of (17) to (24), wherein the neurons are neurons differentiated from human-derived pluripotent stem cells.
(26) The microbead according to (25), wherein the human neurons are neurons differentiated from human iPS cells or human ES cells.
(27) A method for screening a drug for a neurological disease, comprising the following steps of:
   (i) co-culturing neurons differentiated from iPS cells derived from a patient suffering from the neurological disease together with microbeads in which at least one LRRTM molecule selected from the group consisting of the LRRTM (Leucine-rich repeat transmembrane neuronal protein) family molecules or a fusion protein containing the same molecule is fixed to the surface thereof, to induce the formation of the presynaptic apparatus (wherein, the LRRTM molecule or the fusion protein containing the same molecule is fixed to the surface of the microbead via a linker.),
   (ii) adding a target substance into a medium and culturing it, in the step (i) or after the step (i), and
   (iii) then, identifying the effect of the target substance on the formation of the presynaptic apparatus in the neuron.
(28) The method according to (27), wherein the detection is performed by at least one selected from
   (a) observing the morphological state of the presynaptic apparatus formed,
   (b) detecting a neurotransmitter released from the presynaptic apparatus,
   (c) detecting the expression of a protein associated with the release of a neurotransmitter from the presynapse, and
   (d) adding a labeling agent (for example, a dye molecule) that labels synaptic vesicles to a medium, and visualizing and detecting the synaptic vesicles that are taken up after the release of the neurotransmitter from the presynapse.
(29) The method according to (27) or (28), wherein the fusion protein containing the LRRTM molecule is a fusion protein containing the LRRTM molecule and an Fc region of human IgG and the linker is an anti-human IgG Fc antibody fixed to the surface of the microbead, and the fusion protein containing the LRRTM molecule is fixed to the surface of the microbead via a bond between the Fc region of human IgG and the anti-human IgG Fc antibody.
(30) The method according to (27) or (28), wherein the linker is a macromolecule (for example, protein, optionally modified polyethylene glycol, optionally modified sugar chain, optionally modified nucleic acid, etc.), and the length of the linker is 10 nm or more.
(31) The method according to any one of (27) to (30), wherein the LRRTM molecule is LRRTM1, LRRTM2, LRRTM3, or LRRTM4.
(32) The method according to (31), wherein the LRRTM molecule is LRRTM2.
(33) The method according to any one of (27) to (32), wherein the neurological disease is a motor neuron disease, a neuromuscular disease, or a mental disorder.
(34) The method according to any one of (27) to (32), wherein the neurological disease is a neurological disease selected from the group consisting of amyotrophic lateral sclerosis (ALS), spinal muscular atrophy (SMA), severe muscular asthenia, Lambert-Eaton syndrome, Alzheimer's disease, dementia such as frontotemporal dementia, epilepsy, Parkinson's disease, schizophrenia, autism, autism spectrum disorder, and other mental disorders.
(35) A screening method of performing the method as described in any one of (27) to (34) according to high-throughput screening (HTS).
(36) A method for screening a substance that promotes or inhibits the release of a neurotransmitter, comprising the following steps of:
   (a) co-culturing mammalian neurons (preferably, human neurons) together with microbeads in which at least one LRRTM molecule selected from the group consisting of the LRRTM (Leucine-rich repeat transmembrane neuronal protein) family molecules or a fusion protein containing the same molecule is fixed to the surface thereof, to induce the formation of the presynaptic apparatus (wherein, the LRRTM molecule or the fusion protein containing the same molecule is fixed to the surface of the microbead via a linker),
   (b) then, culturing the neurons with a target substance added into a medium, and
   (c) any one step selected from the group consisting of the following detection steps:
      (c-1) detecting a neurotransmitter released into the medium,
      (c-2) detecting the expression of a protein associated with the release of a neurotransmitter from the presynapse, and
      (c-3) adding a labeling agent (for example, a dye molecule) that labels synaptic vesicles to a medium, and visualizing and detecting the synaptic vesicles that are taken up after the release of a neurotransmitter from the presynapse.
(37) The method according to (36), wherein in the step (b), a substance that stimulates the synapse and promotes the neurotransmitter release is further added to a medium.
(38) The method according to (36) or (37), wherein the fusion protein containing the LRRTM molecule is a fusion protein containing the LRRTM molecule and an Fc region of IgG (preferably, human IgG) and the linker is an anti-IgG Fc antibody (preferably, an anti-human IgG Fc antibody) fixed to the surface of the microbead, and the fusion protein containing the LRRTM molecule is fixed to the surface of the microbead via a bond between the Fc region of IgG (preferably, human IgG) and the anti-IgG Fc antibody (preferably, the anti-human IgG Fc antibody).
(39) The method according to (36) or (37), wherein the linker is a macromolecule (e.g., protein, optionally modified polyethylene glycol, optionally modified sugar chain, optionally modified nucleic acid, etc.), and the length of the linker is 10 nm or more.
(40) The method according to any one of (36) to (39), wherein the neurons are human peripheral neurons.
(41) The method according to any one of (36) to (39), wherein the neurons are human central neurons.
(42) The method according to (40), wherein the neurons are human motor neurons.
(43) The method according to (41), wherein the neurons are human glutamatergic neurons.
(44) The method according to any one of (36) to (43), wherein the LRRTM molecule is LRRTM1, LRRTM2, LRRTM3, or LRRTM4.
(45) The method according to (44), wherein the LRRTM molecule is LRRTM2.
(46) The method according to any one of (36) to (45), wherein the neurons are neurons that have been differentiated from human-derived pluripotent stem cells.
(47) The method according to (45), wherein the human neurons are neuron that have been differentiated from human iPS cells or human ES cells.
(48) The method according to any one of (36) to (47), wherein the fusion protein is a fusion protein obtained by expressing, in a host, a plasmid containing a DNA including a DNA which codes the amino acid sequence of the LRRTM molecule and a DNA which codes the amino acid sequence of the Fc region of human IgG.
(49) The method according to (47), wherein the LRRTM molecule is LRRTM2.
(50) A method for diagnosing whether a subject (preferably, a human) is suffering from a disease, comprising
   (I) a step of co-culturing neurons differentiated from iPS cells derived from the subject together with microbeads in which at least one LRRTM molecule selected from the group consisting of the LRRTM (Leucine-rich repeat transmembrane neuronal protein) family molecules or a fusion protein containing the same molecule is fixed to the surface thereof, to induce the formation of the presynaptic apparatus (wherein, the LRRTM molecule or the fusion protein containing the same molecule is fixed to the surface of the microbead via a linker),
   (II) any one detection step selected from the group consisting of the following (a) to (c):
      (a) a step of detecting a neurotransmitter contained in the presynapse or a neurotransmitter released from the presynapse into a medium,
      (b) a step of detecting the expression of a protein associated with the release of a neurotransmitter from the presynapse, and
      (c) a step of adding a labeling agent (for example, a dye molecule) that labels synaptic vesicles to a medium, and visualizing and detecting synaptic vesicles that are taken up after the release of the neurotransmitter from the presynapse; and
   (III) a step of diagnosing the subject as having a disease based on the relationship between the specific disease and the result detected by the detection step.
(51) The method according to (50), wherein the step (II) is (II-1) a step of adding a substance that stimulates the presynapse to induce the release of a neurotransmitter into a medium, and (II-2) a step of detecting the neurotransmitter released from the presynapse into the medium.
(52) The method according to (50) or (51), wherein the fusion protein containing the LRRTM molecule is a fusion protein containing the LRRTM molecule and an Fc region of human IgG and the linker is an anti-IgG Fc antibody (preferably, an anti-human IgG Fc antibody) fixed to the surface of the microbead, and the fusion protein containing the LRRTM molecule is fixed to the surface of the microbead via a bond between the Fc region of human IgG and the anti-IgG Fc antibody (preferably, the anti-human IgG Fc antibody).
(53) The method according to (50) or (51), wherein the linker is a macromolecule (e.g., protein, optionally modified polyethylene glycol, optionally modified sugar chain, optionally modified nucleic acid, etc.), and the length of the linker is 10 nm or more.
(54) The method according to any one of (50) to (53), wherein the neurons are human peripheral neurons.
(55) The method according to any one of (50) to (53), wherein the neurons are human central neurons.
(56) The method according to (54), wherein the neurons are human motor neurons.
(57) The method according to (55), wherein the neurons are human glutamatergic neurons.
(58) The method according to any one of (50) to (57), wherein the LRRTM molecule is LRRTM1, LRRTM2, LRRTM3, or LRRTM4.
(59) The method according to (58), wherein the LRRTM molecule is LRRTM2.

### [Brief Description of Drawings]

Fig. 1 is a view schematically showing a microbead in which an LRRTM2 molecule is fixed to the surface, which is one of the embodiments of the present invention.
Fig. 2 shows the result of the formation of the presynaptic apparatus by co-culturing glutamatergic neurons with LRRTM2-Fc-anti-human IgG Fc antibody microbeads (LRRTM2-Fc Linker (+)) or control Fc-anti-human IgG Fc antibody microbeads (control-Fc Linker (+)).
Fig. 3 shows the result of the formation of the presynaptic apparatus by co-culturing glutamatergic neurons wiht LRRTM2-Fc-anti-human IgG Fc antibody microbeads (LRRTM2-Fc Linker (+)) or LRRTM2-Fc-microbeads (LRRTM2-Fc Linker (-)).
Fig. 4 shows the result of the induction of the synapse formation using LRRTM2-Fc-anti-human IgG Fc antibody microbeads prepared while sequentially reducing the amount of LRRTM2-Fc to be fixed on beads, and the proportion of beads having positive synapsin-inducing activity was shown. 100% is the result of beads prepared using an LRRTM2 solution at a concentration of 50 µg/mL, and 10% is the result of beads prepared using an LRRTM2 solution at a concentration of 1/10 of that. 0% is for the control.
Fig. 5 shows the result of the formation of the presynaptic apparatus by co-culturing motor neurons with LRRTM2-Fc-anti-human IgG Fc antibody microbeads (LRRTM2-Fc Linker (+)) or control Fc-anti-human IgG Fc antibody microbeads (control-Fc Linker (+)).
Fig. 6 shows the result of the formation of the presynaptic apparatus by co-culturing motor neurons with LRRTM2-Fc-anti-human IgG Fc antibody microbeads (LRRTM2-Fc Linker (+)), followed by detecting with the use of a marker VAChT (vesicular acetylcholine transporter).
Fig. 7 shows the result of the formation of the presynaptic apparatus by co-culturing motor neurons with RRTM2-Fc-anti-human IgG Fc antibody microbeads (LRRTM2-Fc Linker (+)) or LRRTM2-Fc-microbeads (LRRTM2-Fc Linker (-)).
Fig. 8 shows the result of the expression of synapsin and acetylcholine transporter (VAChT) after the formation of the presynaptic apparatus from a motor neuron derived from an iPS cell derived from a healthy subject or an ALS patient using the method of the present invention.

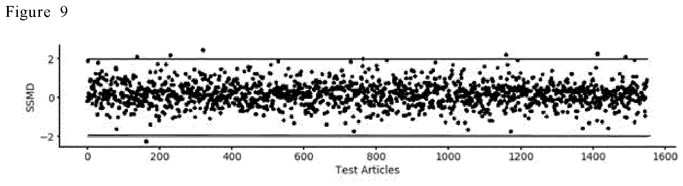

Fig. 9 shows the result of the influences of 1814 compounds listed in the library of FDA-approved drug compounds on the synapse formation by using the screening method of the present invention. LRRTM2-Fc-anti-human IgG Fc antibody microbeads (positive) or control Fc-anti-human IgG Fc antibody microbeads (negative) were cu-cultured with motor neurons to form the presynaptic apparatus in the presence of various compounds, which were then immunostained with a presynaptic apparatus marker (synapsin 1), and the difference in numerical value (SSMD, strictly standardized mean difference) when the fluorescence intensity was compared with the experimental control is shown.

### [Description of Embodiments]

Hereinafter, the present invention will be illustrated with reference to the exemplary embodiments along with preferred methods and materials which can be used in practice of the present invention. Unless otherwise specified in the sentences, any technical terms and scientific terms used in the present specification have the same meaning as those generally understood by those of ordinary skill in the art to which the present invention belongs. Any materials and methods equivalent or similar to those described in the present specification can be used for practicing the present invention. All publications and patents cited herein in connection with the present invention are incorporated by reference, for example, as indicating methodology, materials, etc. that can be used in the present invention. In the present specification, "about" is used to mean that ±10% is allowed.

### (1) Neuron

The neuron that can be used in the method of the present invention are a mammalian neuron. Examples of mammals include primates (e.g., humans), cats, dogs, cows, sheep, goats, horses, rabbits, rats, mice, koalas, and the like, but humans are preferred. The cell that can be used in the method of the present invention are preferably human neurons. Hereinafter, the present invention will be described by taking human neurons as an example, but the present invention is not limited to human neurons. Examples of human neurons include human peripheral neurons and human central neurons, and both of which can be used in the present invention. Human neurons can be used without limitation regardless of their origin. For example, primary culture of cells isolated from humans, cells isolated from humans and established as cell lines, and human neurons differentiated from human-derived pluripotent stem cells can be mentioned, but examples are not limited to these. Preferred are pluripotent stem cells of human origin. The "pluripotent stem cell" used in the present invention refers to a cell having a self-renewal ability, being able to be cultured in vitro, and having a pluripotent ability to differentiate into cells constituting an individual. Specific examples thereof include embryonic stem cells (ES cell), fetal primordial germ cell-derived pluripotent stem cells (GS cell), somatic cell-derived induced pluripotent stem cells (iPS cell) and the like, and the preferably used in the present invention are human-derived iPS cells or ES cells, and particularly preferable are human-derived iPS cells.

In general, ES cells are obtained by culturing fertilized eggs in the blastocyst stage together with feeder cells, separating the cells derived from the proliferated inner cell mass, and repeating the operation of subculture, and finally they can be established as the cell strain. As described above, ES cell are often obtained from fertilized eggs, but can also be obtained from other than fertilized eggs, for example, adipose tissue, placenta, and testis cell, and any ES cell is the subject of the present invention. Methods for producing ES cells from other than fertilized eggs have been reported, and these reports can be appropriately referred to and used. For example, the disclosure of WO2003/046141 can be mentioned, but examples are not limited to this.

iPS cells are artificial stem cells derived from somatic cell and can be produced by introducing specific reprogramming factors into somatic cells in the form of nucleic acids or proteins, which exhibit properties that are almost equivalent to ES cells (e.g., pluripotency of differentiation and proliferative capacity based on self-renewal). Reprogramming factors may be constituted of genes that are specifically expressed in ES cells, their gene products or their non-coding RNAs, genes that play an important role in maintaining undifferentiated ES cells, their gene products or their non-coding RNAs, or low molecular weight compounds. Examples of genes contained in the reprogramming factors are Oct3/4, Sox2, Sox1, Sox3, Sox15, Sox17, Klf4, Klf2, c-MYC, N-Myc, L-Myc, Nanog, Lin28, Fbx15, ERas, ECAT15-2, Tcl1, beta-catenin, Lin28b, Sall1, Sall4, Esrrb, Nr5a2, Tbx3, Glis1 etc. These reprogramming factors may be used alone or in combination. When introducing the c-MYC gene into somatic cells as the reprogramming factor and using it, it is preferable to use an introduction method in which the introduced c-MYC gene is unlikely to be integrated into the chromosome of the target cell after the creation of iPS cells, and examples thereof include, but are not limited to, introduction using a Sendai virus vector or an episomal vector. Many reports have been made on the methods for producing iPS cells, and these reports can be referred to and used by appropriately modifying them. The iPS cell that can be used in the present invention are preferably human-derived iPS cells, for example, human fibroblast-derived iPS cells.

In the present invention, peripheral neurons or central neurons, which are neurons, differentiated from human iPS cells, are preferably used. Many reports have been made on methods for differentiation-inducing a human iPS cell into a peripheral neuron, and these reports can be referred to and used by appropriately modifying them. For methods for differentiation-inducing human iPS cells into peripheral neurons, such as a motor neuron, for example, Chambers, Stuart M., et al. "Highly efficient neural conversion of human ES and iPS cells by dual inhibition of SMAD signaling." Nature biotechnology 27.3 (2009): 275 can be referred to. Many reports have been made on methods for differentiation-inducing a human iPS cell into a central neuron, and these reports can be referred to and used by appropriately modifying them. For the method for differentiation-inducing human iPS cells into central neurons, for example, a glutamatergic neuron, for example, Shi, Yichen, et al. "Human cerebral cortex development from pluripotent stem cells to functional excitatory synapses." Nature neuroscience 15.3 (2012)): 477 can be referred to. Alternatively, various neurons differentiated from human iPS cells are commercially available and can be purchased from, for example, ReproCELL Inc.

Since the formation of the presynaptic apparatus in peripheral neurons, which are neurons, can be examined using the method of the present invention, it becomes possible to evaluate whether a disease of a patient is related to the synapse formation, by using peripheral neurons differentiated from iPS cells derived from a patient with a disease of a peripheral neuron. In addition, it becomes possible to evaluate whether a disease of a patient is related to the function of a synapse (e.g., but not limited to, the ability to release a neurotransmitter) by measuring the functionality of the synapse formed. The diseases of peripheral neurons include, but are not limited to, motor neuron diseases and neuromuscular diseases such as, for example, amyotrophic lateral sclerosis (ALS), spinal muscular atrophy (SMA), severe muscular asthenia, Lambert-Eaton syndrome, and the like.

The human peripheral neurons used in the method of the present invention include, but not limited to, for example, human motor neurons, human sensory neurons, human sympathetic neurons, and human parasympathetic neurons, preferably, human motor neurons.

Since the formation of the presynaptic apparatus in central neurons, which are neurons, can be examined using the method of the present invention, it is possible to evaluate whether a disease of a patient is related to synapse formation by using central neurons differentiated from iPS cells derived from the patient with the disease in a central neuron. In addition, by measuring the functionality of the synapse formed, it is possible to evaluate whether the disease of the patient is related to the synaptic function (e.g., though not limited to, the ability to release a neurotransmitter). The diseases of central neurons include, but are not limited to, for example, dementia such as Alzheimer's disease and frontotemporal dementia, epilepsy, Parkinson's disease, schizophrenia, autism, autism spectrum disorder, and various other mental disorders.

The human central neurons used in the method of the present invention include, but are not limited to, for example, human glutamatergic neurons, cholinergic neurons, adrenalinergic neurons, dopaminergic neurons, serotoninergic neurons, and noradrenalinergic neurons, preferably, human glutamatergic neurons.

According to the method of the present invention, the presynaptic apparatus can be formed using a neuron, and a functional synapse can be formed. Synapse formation can be confirmed, for example, by observing the morphology of the formed synapse or by detecting a protein expressed at the synapse, i.e., a presynaptic apparatus marker (e.g., synapsin 1). Since neurotransmitters are released from functional synapses, it is possible to detect neurotransmitters using the methods of the present invention, and further, the method can also be used for detecting substances that promote or inhibit the release of neurotransmitters. For the ability to release a neurotransmitter from a synapse, a substance actually released from a synapse may be directly detected, or various proteins associated with the release of a neurotransmitter may be detected. The substances released from synapses, i.e., neurotransmitters, include, but are not limited to, for example, amino acids (e.g., glutamic acid, γ-aminobutyric acid, aspartic acid, glycine), peptides (e.g., vasopressin, gastrin, somatostatin, neurotensin, neuropeptide, opioid, secretin, tachykinins), monoamines (e.g., dopamine, noradrenaline, octopamine, tyramine, phenylamine, phenylethanolamine, serotonin, histamine), and acetylcholines. The proteins associated with the release of neurotransmitters from synapses include, but are not limited to, for example, vesicular acetylcholine transporter (VAChT), vesicular glutamate transporter (VGlut), vesicular monoamine transporter (VMAT), vesicular GABA transporter (VGAT), and synaptic vesicle-related molecules such as synapsin, synaptotagmine, synaptophysin, SNAP25, and the like, and it can also be done by detecting the expression of an active band marker such as Bassoon, Piccolo, and the like. In detecting the release of neurotransmitters, synapses may be stimulated as needed, and the synapses release neurotransmitters in response to the stimulation. For example, it is possible that the presynaptic apparatus in a neuron is formed, then, a stimulation is imparted, and a neurotransmitter released into the culture supernatant is detected, using the method of the present invention, but the method is not limited to this. The stimulation is not particularly limited as long as it causes release of a neurotransmitter, and for example, stimulations by various drugs, compounds, electrodes, or the like are mentioned. The detection of a neurotransmitter is not particularly limited, and a method capable of detecting a target substance can be appropriately used. Various methods such as, for example, an ELISA method, various mass spectrometry methods (for example, LC-MS, tandem LC-MS), an enzyme reaction method and the like can be used to detect the target neurotransmitter, but the ELISA method is preferable. This makes it possible to evaluate the functionality of a synapse induced by the method of the present invention, and to screen for compounds that stimulate a synapse to act on the release of a neurotransmitter.

The formation of a functional synapse can also be confirmed by visualizing and detecting a synaptic vesicle that is re-uptaken after the release of a neurotransmitter from the synapse, by using a labeling substance that has been added extracellularly in advance. The labeling substance for a synaptic vesicle includes, but is not limited to, for example, dye molecules, and FM dye (FM^{™} 4-64 Dye (N-(3-triethylammoniumpropyl)-4-(6-(4-(diethylamino)phenyl)hexatrienyl)pyridinium dibromide), available from Thermo Fisher), and the formation of a functional synapse can be confirmed by adding these labeling substances to a medium and culturing the cells.

Using the method of the present invention, it is also possible to select a substance that promotes or inhibits the release of a neurotransmitter under the conditions that mimic the inside of a living body. Examples of the conditions that mimic the inside of a living body include a method of culturing in a state where the axon of a neuron and the synapse formation part can be confirmed as different structures. In carrying out the method of the present invention, various cell culture devices can be used by appropriately improving or modifying them as necessary, but preferably, the device described in WO2017/187696 is used, and by using this device, functional synapse formation can be satisfactorily achieved. Neurotransmitters are released into the culture supernatant by forming functional synapses in a neuron and then stimulating the neuron in the same manner as in vivo using the methods of the invention. The neurotransmitter released into the culture supernatant can be detected and quantified by, for example, mass spectrometry, ELISA, or enzyme reaction method. In such a measurement system, by adding various substances to the medium, substances that promote or inhibit the release of neurotransmitters can be selected. Therefore, the present invention can also be used as a screening method for compounds and the like that affect the release of neurotransmitters under conditions that mimic the inside of a living body.

The neurotransmitter to be detected is not particularly limited, and examples thereof include the above-mentioned acetylcholine, amino acids, monoamines, neuropeptides (polypeptides), and the like. Acetylcholine is released in response to stimuli from synapses at the motor nerve end of the cholinergic nerve. A functional synapse is formed in a neuron, and then, the neuron is stimulated (e.g., by adding glutamic acid, high concentration potassium, 4-aminopyrrolidone, etc.), thereby, acetylcholine is released into the culture supernatant, by using the method of the present invention, but it is not limited to this. Acetylcholine released into the culture supernatant can be detected and quantified by, for example, mass spectrometry, ELISA, or an enzymatic reaction method using cholinesterase. The ELISA method is preferable. In addition, by adding various substances to the medium, substances that promote or inhibit the release of acetylcholine can be selected. For example, the related evaluation method includes evaluation of an acetylcholine-based neurotransmission improving agent, evaluation of potassium ion channel or calcium ion channel inhibitor or activator, but the method is not limited to them.

According to the method of the present invention, a functional synapse can be formed using a neuron, and a neurotransmitter is released from the synapse, therefore, the method of the present invention can be used to detect an abnormality in the release of a neurotransmitter. Therefore, it is possible to diagnose a disease by using the method of the present invention based on the relationship between the release of a specific neurotransmitter and a specific disease. For example, after differentiation-inducing iPS cells derived from a target patient into a neuron, synapses can be formed according to the present invention using the neuron. Then, by detecting a specific neurotransmitter (e.g., the neurotransmitter described above) released from a synapse, or by detecting the expression in a synapse of a protein associated with the release of a specific neurotransmitter (e.g., the above-described protein), or by visualizing and detecting a synapse vesicle that is re-uptaken after the release of a neurotransmitter from a synapse by using a labeling substance (for example, a dye molecule) that has been added extracellularly in advance, it becomes possible to judge whether the subject has an abnormality in the synapse formation or suffers from a specific disease associated with the release of the neurotransmitter. Therefore, the present invention is also a method for diagnosing a disease based on any of the above detection methods.

### (2) LRRTM (Leucine-rich repeat transmembrane neuronal protein) molecule

The LRRTM molecule used in the present invention is a molecule called the LRRTM family. The LRRTM family is one of the postsynaptic terminal side synaptic organizer molecular families, and four types have been reported in humans: LRRTM1, LRRTM2, LRRTM3, and LRRTM4. In the present invention, any of these four types of LRRTM molecules can be used, but LRRTM2 is preferable. The LRRTM molecule, which is one of the synaptic adhesion molecules, is a single-pass transmembrane protein and contains an extracellular domain and an internal domain. The LRRTM molecule that can be used in the present invention is a molecule that contains at least an extracellular domain. Thus, for example, in the present invention, the human LRRTM1 molecule means a human LRRTM1 molecule having at least 1-st to 392-nd amino acid sequence of SEQ ID NO: 1 which is the extracellular domain.

The human LRRTM1 is a membrane protein consisting of 488 amino acids represented by SEQ ID NO: 1. After translation, it is expressed as a protein consisting of 522 amino acids having a signal peptide consisting of 34 amino acids. The extracellular domain of LRRTM1 has been reported to be the 1-st to 392-nd of the amino acid sequence represented by SEQ ID NO: 1, and this region is considered to be important for the induction of the formation of the presynaptic apparatus. The LRRTM1 molecule used in the present invention is not limited to the protein having the 488 amino acid sequence represented by SEQ ID NO: 1, and means a protein containing the 1-st to 392-nd amino acids of the amino acid sequence represented by SEQ ID NO: 1 having the activity of inducing the formation of the presynaptic apparatus, and may be those in which a part (e.g., 10%, 9%, 8%, 7%, 6%, 5%, 4%, 3%, 2% or 1% or less of the entire sequence) is substituted/deleted or added in their sequences, as long as they have the activity of inducing the formation of the presynaptic apparatus of a neuron.

The human LRRTM2 is a membrane protein consisting of 483 amino acids represented by SEQ ID NO: 2. After translation, it is expressed as a protein consisting of 516 amino acids having a signal peptide consisting of 33 amino acids. The extracellular domain of LRRTM2 has been reported to be the 1-st to 389-th of the amino acid sequence represented by SEQ ID NO: 2, and this region is considered to be important for the induction of the formation of the presynaptic apparatus. The LRRTM2 molecule used in the present invention is not limited to the protein having the 483 amino acid sequence represented by SEQ ID NO: 2, and means a protein containing the 1-st to 389-th amino acids of the amino acid sequence represented by SEQ ID NO: 2 having the activity of inducing the formation of the presynaptic apparatus, and may be those in which a part (e.g., 10%, 9%, 8%, 7%, 6%, 5%, 4%, 3%, 2% or 1% or less of the entire sequence) is substituted/deleted or added in their sequences, as long as they have the activity of inducing the formation of the presynaptic apparatus of a neuron.

The human LRRTM3 is a membrane protein consisting of 551 amino acids represented by SEQ ID NO: 3. After translation, it is expressed as a protein consisting of 581 amino acids having a signal peptide consisting of 30 amino acids. The extracellular domain of LRRTM3 has been reported to be the 1-st to 389-th of the amino acid sequence represented by SEQ ID NO: 3, and this region is considered to be important for the induction of the formation of the presynaptic apparatus. The LRRTM3 molecule used in the present invention is not limited to the protein having the 551 amino acid sequence represented by SEQ ID NO: 3, and means a protein containing the 1-st to 389-th amino acids of the amino acid sequence represented by SEQ ID NO: 3 having the activity of inducing the formation of the presynaptic apparatus, and may be those in which a part (e.g., 10%, 9%, 8%, 7%, 6%, 5%, 4%, 3%, 2% or 1% or less of the entire sequence) is substituted/deleted or added in their sequences, as long as they have the activity of inducing the formation of the presynaptic apparatus of a neuron.

The human LRRTM4 is a membrane protein consisting of 560 amino acids represented by SEQ ID NO: 4. After translation, it is expressed as a protein consisting of 590 amino acids having a signal peptide consisting of 30 amino acids. The extracellular domain of LRRTM4 has been reported to be the 1-st to 394-th of the amino acid sequence represented by SEQ ID NO: 4, and this region is considered to be important for the induction of the formation of the presynaptic apparatus. The LRRTM4 molecule used in the present invention is not limited to the protein having the 560 amino acid sequence represented by SEQ ID NO: 4, and means a protein containing the 1-st to 394-th amino acids of the amino acid sequence represented by SEQ ID NO: 4 having the activity of inducing the formation of the presynaptic apparatus, and may be those in which a part (e.g., 10%, 9%, 8%, 7%, 6%, 5%, 4%, 3%, 2% or 1% or less of the entire sequence) is substituted/deleted or added in their sequences, as long as they have the activity of inducing the formation of the presynaptic apparatus of a neuron.

### (3) Fusion protein

The fusion protein containing the LRRTM molecule used in the present invention is a fusion protein containing any of the above-mentioned LRRTM molecules. The protein portion of the fusion protein other than the LRRTM molecule can be arbitrarily selected as long as it does not inhibit the formation of the presynaptic apparatus, which is the object of the present invention. By using a fusion protein, it is possible to provide a bond to a linker and a distance between the LRRTM molecule and the surface of the microbead, and to provide motility of the LRRTM molecule in a three-dimensional space, though not limited to these. The fusion protein is preferably a fusion protein containing the LRRTM molecule and an Fc region of human IgG. Therefore, the preferred fusion protein used in the present invention is a fusion protein containing at least one of these four types of LRRTM molecules and an Fc region of human IgG, and particularly preferably, a fusion protein containing the above-mentioned human LRRTM2 (Leucine-rich repeat transmembrane neuronal protein 2) molecule and an Fc region of human IgG.

Hereinafter, the fusion protein containing an Fc region of human IgG and the LRRTM molecule will be described by taking the LRRTM2 molecule as an example, but it can be easily understood by those skilled in the art that the same shall apply to other LRRTM molecules.

The fusion protein composed of an Fc region of human IgG and the LRRTM2 molecule can be produced by appropriately referring to a known method. For example, it can be produced by preparing a vector containing a DNA encoding the amino acid sequence of 483 amino acids of human LRRTM2 and a DNA encoding the amino acid sequence of the Fc region of human IgG, and transforming and expressing it in any host used for expressing a recombinant protein, such as, Escherichia coli, yeasts, insect cells, cultured mammalian cells, and the like, but the method is not limited to this. When the sugar chain modification is desired, a host in which a sugar chain-modified recombinant protein can be produced can be appropriately selected and the protein can be produced according to a conventional method. The connection between DNA that codes human LRRTM2 and DNA that codes an Fc region of human IgG can be attained using a conventional method, and any peptide sequence can be inserted therebetween in connecting them. In addition, the order of both DNAs may be either first as long as having the inducing activity when the obtained fusion protein is fixed to a microbead, and the fusion protein to be expressed may be in the order of human LRRTM2-(any peptide sequence)-human IgG Fc region or in the order of human IgG Fc region-(any peptide sequence)-human LRRTM2, viewed from the N-terminal. By purifying the obtained fusion protein using a known method, a fusion protein containing the LRRTM molecule used in the present invention can be obtained.

Fusion proteins composed of human LRRTM2 and an Fc region of human IgG are also commercially available, and can be used. For example, they are sold by R & D Systems.

In addition to the LRRTM2 molecule and an Fc region of human IgG, the fusion protein can contain any peptides connecting the LRRTM2 molecule and an Fc region of human IgG (for example, but not limited to, peptides composed of 1 to 100, 3 to 80, 5 to 80, 5 to 50, 5 to 30, 5 to 20 amino acids), any peptides constituting the N-terminal (for example, but not limited to, peptides composed of 1 to 50, 1 to 30, 2 to 20, 2 to 10 amino acids), and any peptides constituting the C-terminal (for example, but not limited to, peptides composed of 1 to 50, 1 to 30, 2 to 20, 2 to 10 amino acids).

### (4) Linker

As the linker that can be used in the present invention, any substance can be used as long as it can link between an LRRTM molecule or a fusion protein containing the same and a microbead. Examples thereof include macromolecules such as proteins, polyethylene glycol (PEG) which may be modified (optionally modified PEG), sugar chains which may be modified (optionally modified sugar chains), nucleic acids which may be modified (optionally modified nucleic acids), and the like, but proteins and PEG which may be modified are preferred. PEG which may be modified includes those in which any substituent is bonded to any position of PEG. Many reports have been made on the modification of PEG, and these can be referred to as appropriate. The sugar chains which may be modified include those in which any substituent is bonded to any position of the sugar chain. Many reports have been made on the method for modifying the sugar chain, and these can be referred to as appropriate. The type of the sugar chain is not particularly limited, and is, for example, a sugar chain constituted of one or a plurality types of arbitrary sugars selected from sugars of 5 monosaccharides or 6 monosaccharides, and the sugar chain may be linear or branched. As the nucleic acid, any of DNA and RNA can be used, and the ribonucleotide/ribonucleoside that constitutes DNA/RNA may be modified.

By using a linker, it is possible to fix an LRRTM molecule to a microbead, or to provide a distance between an LRRTM molecule and the surface of a microbead, thereby providing the motility of an LRRTM molecule in a three-dimensional space, but it is not limited to this. Therefore, the linker preferably has a reactive group and has a certain length. The length of the linker is preferably 10 nm or more, more preferably 15 nm or more, still more preferably 20 nm or more. Furthermore, for the induction of the formation of the presynaptic apparatus, it is desired that a certain number or more of LRRTM molecules are fixed to the surface of a microbead, therefore, a linker capable of bonding to a plurality of LRRTM molecules or to a fusion protein containing a plurality of LRRTM molecules is preferably used.

### (5) Bond with linker

The bond between an LRRTM molecule and the linker can be carried out by appropriately referring to a known method. For example, this can be done by chemically bonding the end of the LRRTM molecule or the side chain of the amino acids constituting the molecule to the linker. For example, an amine or carboxyl group can be chemically bonded by reacting with a reactive group present in the linker. When the linker is a protein, known methods used for bonding proteins together can be used. In the case where the linker is PEG that may be modified, known methods of PEG-modifying the protein can be used. When the linker is a sugar chain, it can be bonded, for example, by reacting an aldehyde group obtained by oxidizing the sugar chain with a group present in the LRRTM molecule, for example, an amine.

The bond between the fusion protein containing an LRRTM molecule and the linker can be carried out by appropriately referring to a known method depending on the type of the fusion protein and the type of the linker. As a method for chemically bonding the fusion protein and the linker, the above-described methods can be mentioned. Further, for example, when the fusion protein contains an LRRTM molecule and an Fc region of human IgG, the Fc region of IgG and the linker can be chemically bonded, and additionally, it is also possible to use a human anti-IgG Fc antibody as the linker and to bond the fusion protein and the linker (anti-IgG Fc antibody) by an antigen-antibody reaction.

### (6) Microbead

The microbead that can be used in the present invention can be used without particular limitation as long as they do not particularly affect the survival of a neuron when cultured with a neuron, and for example, microbeads consisting of polyethylene, silica, or magnetic beads can be mentioned. The size of the microbead is not particularly limited, but for example, the average diameter is 1 to 50 µm, preferably 5 to 30 µm, more preferably 5 to 20 µm, still more preferably 5 to 15 µm, and most preferably about 10 µm.

In the microbead used in the present invention, an LRRTM molecule or a fusion protein containing the same molecule is fixed to the surface via a linker. Bonding of the microbead surface and the linker can be performed by a conventional method depending on the type of the linker. For example, when a microbead on which the protein A is fixed is used, it is possible to use a protein as a linker and to bond the linker by a conventional method. When a microbead on which streptavidin is fixed is used, it is possible to bond a linker to the microbead by using a biotin-modified linker. Alternatively, fixation is possible also by chemically modifying the surface of a microbead and introducing a reactive group, and reacting it with a linker. The chemical modification method for introducing a reactive group onto the surface of the microbead can be carried out with reference to a known method. In addition, the reaction between the microbead into which the reactive group has been introduced and the linker can also be carried out according to a conventional method. For example, when a protein is used as a linker, an amine or carboxyl group present in the protein is reacted with a reactive group introduced on the bead surface (for example, a carboxyl group or amine). In addition, methods used in the technical field of peptide synthesis can be appropriately used. When a sugar chain is used as the linker, for example, an aldehyde group obtained by oxidizing the sugar chain can be reacted with a reactive group (for example, an amine) introduced into the surface of the beads.

In one embodiment of the microbead used in the present invention, an anti-human IgG Fc antibody is bonded to the surface thereof. The bond of an anti-human IgG Fc antibody to the surface of the microbead can be performed according to a conventional method. For example, by treating a biotinylated anti-human IgG Fc antibody by a conventional method using a streptavidin-coated microbead, it is possible to prepare a microbead on which an anti-human IgG Fc antibody is fixed to the surface owing to a biotin-avidin bond, but it is not limited to this. Alternatively, it is possible to prepare a microbead in which an anti-human IgG Fc antibody is fixed to the surface by introducing a reactive group having reactivity with a protein (amino acid) into the surface of a microbead, then, reacting with an anti-human IgG Fc antibody, according to a conventional method. Furthermore, it is possible to prepare a microbead in which an anti-human IgG Fc antibody is fixed to the surface by treating an anti-human IgG Fc antibody according to a conventional method using a protein A or G-coated microbead.

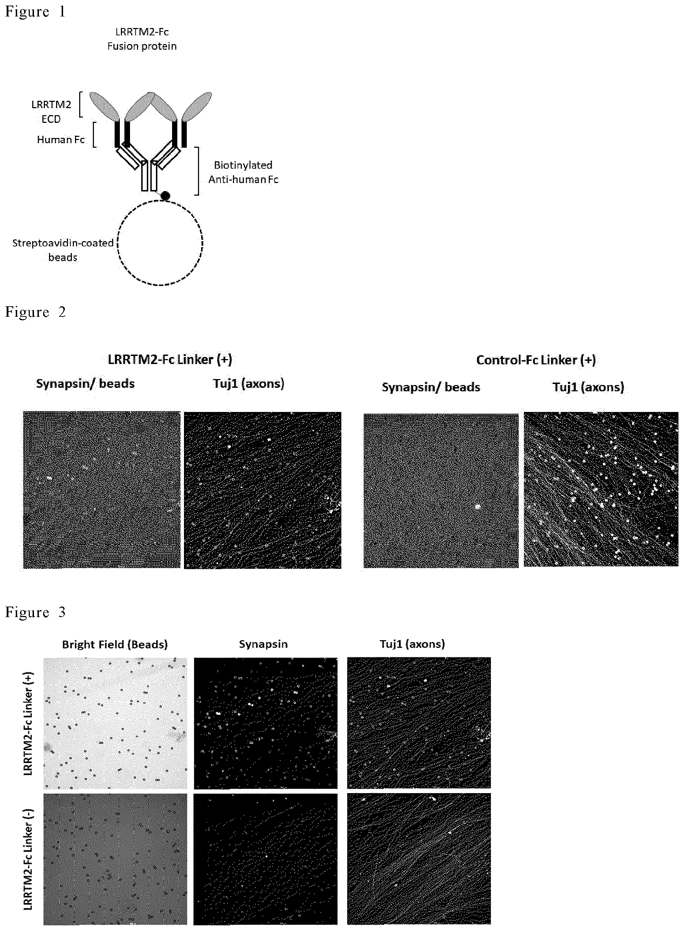

Fixation of a fusion protein composed of an LRRTM molecule and IgG Fc onto the surface of the microbead, which is one embodiment of the present invention, can be carried out by an antigen-antibody reaction between an anti-human IgG Fc antibody fixed on the surface of the microbead and the Fc region of human IgG of the fusion protein. This makes it possible to prepare a microbead in which an LRRTM molecule is fixed to the surface. Fig. 1 is a schematic representation of the microbead in which an LRRTM2 molecule is fixed to the surface. In this embodiment, four LRRTM molecules can be fixed at the same location on the surface of the microbead.

One embodiment of the present invention is a method for inducing the formation of the presynaptic apparatus in a neuron using the microbead in which an LRRTM molecule is fixed to the surface prepared as described above.

Induction of the formation of the presynaptic apparatus (hereinafter, may be simply referred to as synapse formation induction) can be performed by co-culturing a neuron with the microbead of the present invention. The culture can be carried out by appropriately referring to the reported culture conditions of various neurons. For example, motor neurons differentiated from human iPS cells are cultured, and the cells are seeded on a matrix-coated 96-well plate. Alternatively, the differentiated motor neurons can be cultured to produce neurospheres, and the prepared neurospheres can be seeded one by one in each well. Preferable is a method using neurospheres. Then, after culturing in a neuron culture medium for a certain period of time, the microbead of the present invention in which an LRRTM molecule is fixed to the surface is added to each well, cultured continuously, to induce the formation of the presynaptic apparatus. The concentration of the microbead added to each well is not particularly limited and can be appropriately selected according to the experimental conditions.

Confirmation of the formation of the presynaptic apparatus can be performed by detecting the expression of the presynaptic apparatus marker. The marker includes, but not limited to, for example, synapsin, synaptophysin, synaptobrevin, neurotransmitter transporters (VAChT, VGlut1, VGlut2, etc.), SNAP25, Bassoon, Piccolo, etc., but preferably, synapsin and synaptophysin. The marker can be detected, for example, by using an antibody against each marker and using an immunostaining method.

Confirmation of the formation of the presynaptic apparatus can also be performed by detecting neurotransmitters released from synapses. The neurotransmitter to be detected is not particularly limited and can be arbitrarily selected according to the purpose. Examples thereof include, but not limited to, acetylcholine, amino acids, monoamines, neuropeptides (polypeptides), and the like. By detecting and measuring these neurotransmitters, it is also possible to evaluate the functionality of synapses including the presynaptic apparatus derived from a neuron using the method of the present invention.

Another embodiment of the present invention is a microbead in which an LRRTM molecule is fixed to the surface prepared as described above, which can be used for culturing a neuron to induce the formation of a synapse. The use of such a microbead can be carried out according to the above descriptions.

Another embodiment of the present invention is a method for screening drugs for a neurological disease, using neurons differentiated from iPS cells derived from a patient suffering from the neurological disease, by adding a target substance to a medium and culturing the neurons, in inducing or after inducing the formation of the presynaptic apparatus, neuron, with the use of a microbead in which an LRRTM molecule is fixed to the surface prepared as described above.

Another embodiment of the present invention is a method for screening substances that promote or inhibit the release of a neurotransmitter, using a synapse derived from a neuron, by using a microbead in which an LRRTM molecule is fixed to the surface prepared as described above.

Another embodiment of the present invention is a method for diagnosing a subject as having a specific disease based on the relation between a specific neurotransmitter and the specific disease, by detecting the specific neurotransmitter released from a synapse into a medium or detecting the expression of a protein involved in the release of the specific neurotransmitter from a synapse, after inducing the formation of the presynaptic apparatus using neurons differentiated from iPS cells derived from the target human, with the use of a microbead in which an LRRTM molecule is fixed to the surface prepared as described above.

### [Examples]

Hereinafter, the present invention will be specifically described with reference to examples, but the present invention is not limited to the following examples.

### Cell types and medium

Human iPS-induced glutamatergic neurons and human iPS-induced motor neurons were purchased from CDI (Cellular Dynamics International, Inc., USA). As the medium, Neurobasal plus medium, B27 plus supplement (Thermo Fischer Scientific) was used, and 20 µg/ml BDNF, 20 µg/ml GDNF, and penicillin/streptomycin were added.

### (Example 1) Activated microbead and control bead

### (1) Preparation of LRRTM2-Fc-anti-human IgG Fc antibody microbead

Streptavidin coated microbeads (Bangs Laboratories, Inc; made of polystyrene, average diameter 9.94 µm) were washed twice with a wash buffer (PBS, 0.01% BSA, 0.05% Triton X-100), and reacted with a biotinylated anti-human IgG (Fc specific) antibody (Sigma-Aldrich Company; mouse monoclonal antibody) in a binding buffer (PBS, 0.01% BSA), thereby fixing the biotinylated anti-human IgG (Fc specific) antibody to the streptavidin coated microbeads. The beads washed three times with a wash buffer were used as the streptavidin-anti-human IgG Fc antibody beads.

Next, the streptavidin-anti-human IgG Fc antibody beads were suspended in a binding buffer, and a fusion protein (LRRTM2-Fc; R & D systems) composed of the extracellular domain of LRRTM2 (1-st to 389-th of the amino acid sequence represented by SEQ ID NO: 2) and an Fc portion of human IgG was added therein, thereby fixing LRRTM2-Fc to the streptavidin-anti-human IgG Fc antibody beads. After the reaction, it was washed with a wash buffer and suspended in a binding buffer. This was used as an LRRTM2-Fc-anti-human IgG Fc antibody microbead suspension.

### (2) Fc-anti-human IgG Fc antibody microbead

An Fc portion of human IgG (Native human IgG Fc fragment protein; Abcam) was added instead of the fusion protein composed of the extracellular domain of LRRTM2 and an Fc portion of human IgG, and microbeads were prepared in the same manner as described above.

### (3) LRRTM2-Fc-microbead

Protein A-coated microbeads (Bangs Laboratories, Inc; made of polystyrene, average diameter 9.94 µm) were washed with a wash buffer (PBS, 0.01% BSA, 0.05% Triton X-100) twice, then, suspended in a binding buffer, and after that, LRRTM2-Fc (R & D systems) was added to prepare beads in which the LRRTM2-Fc fusion protein was directly fixed to the protein A-coated microbeads. After washing the microbeads three times, they were suspended in a binding buffer to prepare an LRRTM2-Fc-microbead suspension.

### (4) Fc-microbead

An Fc protein without the LRRTM2 portion (Native human IgG Fc fragment protein; Abcam) was used instead of the LRRTM2-Fc fusion protein, and microbeads were prepared in the same manner as described in (3) above.

### (Example 2)

The activity of inducing the formation of the presynaptic apparatus in a human glutamatergic neuron by LRRTM2 was examined as follows.

The human iPS-induced glutamatergic neurons were purchased from CDI. According to the CDI protocol, neurospheres containing 4 x 10⁴ cell were prepared, cultured for 2-3 days, and then seeded one by one on a matrix-coated 96-well plate. The neurospheres were cultured in a neuron medium (Neurobasal plus medium, B27 plus supplement, 20 µg/ml BDNF, 20 µg/ml GDNF, penicillin/streptomycin) for 7-10 days, then, an LRRTM2-Fc-anti-human IgG Fc antibody microbead or Fc-anti-human IgG Fc antibody microbead (negative control) suspension was added in an amount of 0.5 µL for each well, and co-cultured for 20-48 hours. Thereafter, the co-cultured cells were fixed with 2% PFA, the cell membrane was permeation-treated with a surfactant and blocking was performed, then, immunostaining with the presynaptic apparatus marker (synapsin) was performed. In addition, immunostaining with betaIII tubulin antibody was also performed at the same time to visualize neurites. The following antibodies were used for immunostaining: Anti-betaIII tubulin (Tuj1), mouse monoclonal (marker for neurite); Anti-synapsin (synaptic systems, Inc.), rabbit polyclonal (marker for presynaptic apparatus). Blocking Buffer (PBS + 2% Normal Goat Serum + 1% BSA + 0.02% Triton X-100) was used for blocking.

As a result, synapsin accumulation was induced at the site of contact between the human iPS-induced glutamatergic nerve axon and the LRRTM2-Fc-anti-human IgG Fc antibody microbead, while the accumulation was not found on the negative control Fc-anti-human IgG Fc antibody microbead. The results are shown in Fig. 2. This experiment demonstrates that the extracellular domain of LRRTM2 has the activity of inducing the formation of the presynaptic apparatus in a human iPS-induced glutamatergic neuron.

### (Example 3)

Next, the relationship between the method of fixing LRRTM2 on a microbead and the activity of inducing the formation of the presynaptic apparatus was tested.

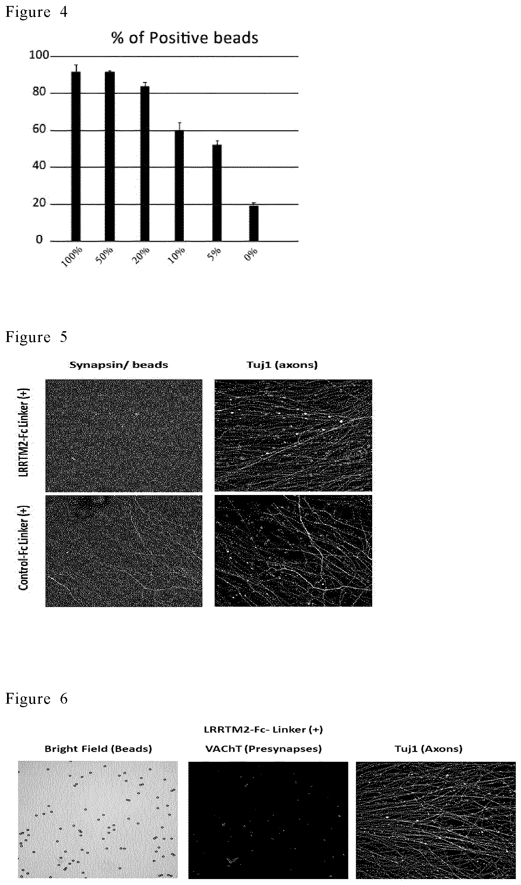

LRRTM2-Fc-anti-human IgG Fc antibody microbeads, Fc-anti-human IgG Fc antibody microbeads (negative control), LRRTM2-Fc-microbeads and Fc-microbeads (negative control) were each co-cultured with human iPS-induced glutamatergic neurons, and in the same manner as in Example 2, immunostained with synapsin, then, the activities of inducing the formation of the presynaptic apparatus were compared. As a result, a high frequency of clear synapsin accumulation was observed at the site of contact between the human iPS-induced glutamatergic nerve axon and LRRTM2-Fc-anti-human IgG Fc antibody microbead, while clear accumulation of synapsin was not observed at the site of contact with the LRRTM2-Fc-microbead. The results of immunostaining are shown in Fig. 3. In addition, synapsin accumulation did not occur on the respective negative control Fc-anti-human IgG Fc antibody microbead or Fc-microbead. These results suggest that the method of fixing LRRTM2-Fc on a microbead is important for giving the activity of inducing the formation of the presynaptic apparatus in an iPS-induced glutamatergic neuron. In addition, when fixing was performed while sequentially diluting the concentration of LRRTM2-Fc in the solution to be added, that is, when the amount of LRRTM2-Fc to be fixed on beads was sequentially reduced, in fixing LRRTM2-Fc to microbeads to prepare LRRTM2-Fc-anti human IgG Fc antibody microbeads, a remarkable decrease in the activity was observed from a certain stage, and it could be confirmed that a certain level or more of fixation of LRRTM-2 was important, and it was confirmed that the proportion of beads with positive synapsin-inducing activity decreased with the decrease in the fixation amount. The results are shown in Fig. 4. This suggests that the number of molecules and the motility of molecules when LRRTM2-Fc is fixed to microbeads may be important for the acquisition of the activity for inducing the formation of the presynaptic apparatus.

### (Example 4)

Similar studies were conducted on the formation of the presynaptic apparatus in human motor neuron.

Human iPS-induced motor neurons were purchased from CDI. According to the CDI protocol, neurospheres containing 2 x 10⁴ cells were prepared, cultured for 2-3 days, and then seeded one by one on a matrix-coated 96- well plate. The neurospheres were cultured on a neuron medium (Neurobasal plus medium, B27 plus supplement, 20 µg/ml BDNF, 20 µg/ml GDNF, penicillin/streptomycin) for 10-21 days, then, an LRRTM2-Fc-anti-human IgG Fc antibody microbead or Fc-anti-human IgG Fc antibody microbead (negative control) suspension was added in an amount of 0.5 µL for each well, and co-cultured for 20-48 hours. Immunostaining was performed in the same manner as in the above experiment, but VAChT (vesicular acetylcholine transporter) was also used in addition to synapsin as the presynaptic apparatus marker. Anti-VAChT rabbit polyclonal antibody (synaptic systems, Inc.) was used.

As a result, synapsin was strongly accumulated at the site of contact between the human iPS-induced motor nerve axon and the LRRTM2-Fc-anti-human IgG Fc antibody microbead, while synapsin did not accumulate at the site of contact with the negative control Fc-anti-human IgG Fc antibody microbead. The results are shown in Fig. 5. Furthermore, as shown in Fig. 6, accumulation of VAChT was observed at the site of contact between the human iPS-induced motor nerve axon and the LRRTM2-Fc-anti-human IgG Fc antibody microbead, while the accumulation was not observed at the site of contact with the negative control bead. These results demonstrate that the extracellular domain of LRRTM2 has the activity of inducing the formation of the cholinergic presynaptic apparatus in human motor neurons. This fact is a very valuable and novel discovery in the synapse formation mechanism of a motor neuron, which has been poorly known so far.

### (Example 5)

Regarding the activity of inducing the formation of the cholinergic presynaptic apparatus in a motor neuron, the relationship with the method for fixing LRRTM2 on a microbead was examined in the same manner as in Example 3.

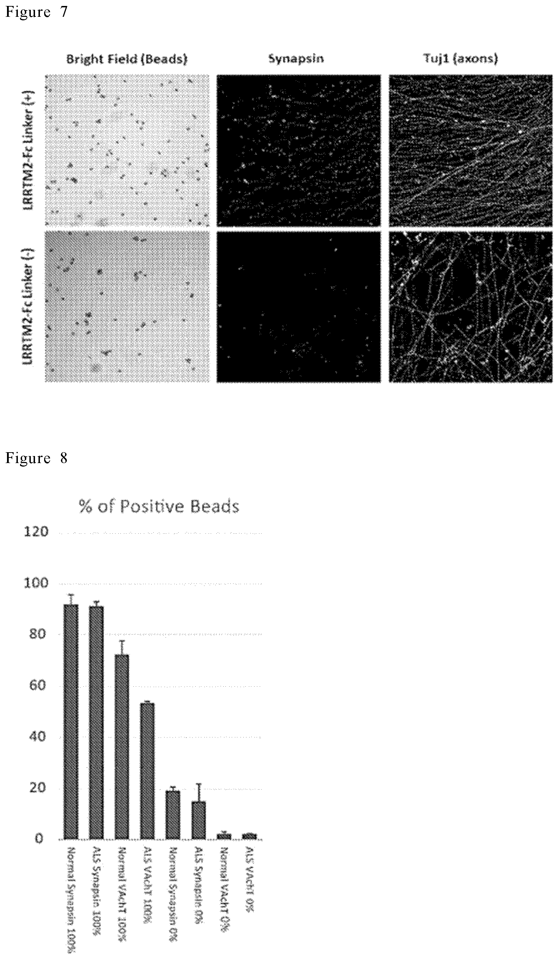

LRRTM2-Fc-anti-human IgG Fc antibody microbeads, Fc-anti-human IgG Fc antibody microbeads (negative control), LRRTM2-Fc-microbeads and Fc-microbeads (negative control) were each co-cultured with human iPS-induced motor neurons. Thereafter, immunostaining was performed, and formations of the presynaptic apparatus were compared. As a result, a high frequency of clear synapsin and VAChT accumulation was observed at the site of contact between the human iPS-induced motor nerve axon and the LRRTM2-Fc-anti-human IgG Fc antibody microbead, while clear accumulation of synapsin or VAChT was not observed at the site of contact with the LRRTM2-Fc-microbead. The results are shown in Fig. 7. In addition, accumulation of synapsin and VAChT did not occur also on the negative control Fc-anti-human IgG Fc antibody microbead or Fc-microbead.

These results suggest that the method of fixing LRRTM2-Fc onto a microbead is important for acquiring the activity for inducing the formation of the cholinergic presynaptic apparatus even in iPS-induced motor neurons.

### (Example 6)

Synapse induction from a motor neuron derived from healthy subjects and ALS patients was performed as follows.

Healthy human iPS-induced motor neurons and ALS patient human iPS-induced motor neurons were purchased from iXCells Biotechnologies. The formation of the presynaptic apparatus of human motor neurons was performed in the same manner as in Example 4. However, since the iPS-induced motor neurons were purchased from iXCells Biotechnologies in this example, the neurospheres were prepared according to the protocol of iXCells Biotechnologies.

Immunostaining was performed to identify the neurotransmitters contained in the presynaptic apparatus induced as described above. Synapsin was used as the marker for all presynaptic apparatus, and all the presynaptic apparatus were detected. On the other hand, VAChT was used as the marker for the cholinergic presynaptic apparatus, and synaptic vesicles in which acetylcholine as a neurotransmitter is accumulated were detected. As a result, the synapsin-positive presynaptic apparatus was induced to the same extent from the motor neurons of healthy subjects and ALS patients, while the positive rate of VAChT of the presynaptic apparatus induced from the motor neurons of ALS patients was significantly lower than that of motor neurons of healthy subjects (Fig. 8). This suggests that a motor neuron derived from ALS patients have a low ability to release acetylcholine. Therefore, it is suggested that the motor neurons of healthy subjects and ALS patients can be discriminated by detecting the amount of acetylcholine released in response to stimulation of a neuron.

### (Example 7)

Using the synapses derived from the motor neurons of ALS patients and the synapses derived from the motor neurons of healthy subjects prepared by the method of Example 6, the amounts of acetylcholine released from the synapses can be compared as follows.

Glutamic acid is added as an inducer of acetylcholine release to a medium containing the cell bodies of motor neurons in which synapses are induced, to induce the release of acetylcholine. After culturing for several days, the medium is collected, the amount of acetylcholine in the medium is measured by ELISA, and the acetylcholine release abilities of synapses derived from ALS patients and synapses derived from healthy subjects can be compared.

### (Example 8)

The method of the present invention was used to screen for compounds that affect the synapse formation.

The formation of synapsin was tested in the same manner as in Example 2 using 1814 kinds of compounds listed in the library of FDA-approved drug compounds. Each compound to be evaluated was added together with a microbead to a final concentration of 10 µM. When the presynaptic apparatus marker (synapsin 1) was immunostained and the fluorescence intensity on the microbead was measured for evaluation, 2 or more SSMD (strictly standardized mean difference) were detected in 7 compounds, thus, significant promotion of the synapse formation were detected.

The foregoing merely illustrates objects and subjects of the present invention, and is not intended to be limiting the accompanying Claims. Without departing from the accompanying Claims, various modifications and alterations to the described embodiments will be apparent to those skilled in the art in view of the teachings herein.

### [Industrial Applicability]

The method and the microbead of the present invention are useful as tools for inducing synapse formation of a neuron, and can also be used for screening methods.

## Claims

1. A method for inducing the formation of the presynaptic apparatus in human neurons, comprising co-culturing the neurons with microbeads in which at least one LRRTM molecule selected from the group consisting of LRRTM (Leucine-rich repeat transmembrane neuronal protein) family molecules or a fusion protein containing the same molecule is fixed to the surface thereof,
wherein the LRRTM molecule or the fusion protein containing the same molecule is fixed to the surface of the microbead via a linker.

2. The method according to claim 1, wherein the fusion protein containing the LRRTM molecule is a fusion protein containing the LRRTM molecule and an Fc region of human IgG and the linker is an anti-human IgG Fc antibody fixed to the surface of the microbead, and the fusion protein containing the LRRTM molecule is fixed to the surface of the microbead via a bond between the Fc region of human IgG and the anti-human IgG Fc antibody.

3. The method according to claim 1, wherein the linker is a macromolecule selected from the group consisting of protein, optionally modified polyethylene glycol, optionally modified sugar chain, and optionally modified nucleic acid, and the length of the linker is 10 nm or more.

4. The method according to claim 1, wherein the distance between the LRRTM molecule or the LRRTM molecule which is a part of the fusion protein fixed to the surface of the microbead, and the surface of the microbead, is 10 nm or more.

5. The method according to any one of claims 1 to 4, wherein the human neurons are human peripheral neurons.

6. The method according to any one of claims 1 to 4, wherein the human neurons are human central neurons.

7. The method according to claim 5, wherein the human neurons are human motor neurons.

8. The method according to claim 6, wherein the human neurons are human glutamatergic neurons.

9. The method according to any one of claims 1 to 8, wherein the LRRTM molecule is an LRRTM 1 molecule, an LRRTM2 molecule, an LRRTM3 molecule, or an LRRTM4 molecule.

10. The method according to claim 9, wherein the LRRTM molecule is LRRTM2.

11. The method according to any one of claims 1 to 10, wherein the neurons are human-derived pluripotent stem cells.

12. The method according to claim 11, wherein the neurons are human iPS cells or human ES cells.

13. The method according to any one of claims 1 to 12, wherein the fusion protein is a fusion protein obtained by expressing, in a host, a plasmid containing a DNA including a DNA which codes the amino acid sequence of the LRRTM molecule and a DNA which codes the amino acid sequence of the Fc region of human IgG.

14. The method according to claim 13, wherein the LRRTM molecule is LRRTM2.

15. The method according to any one of claims 1 to 14, further comprising a step of immunostaining the presynaptic apparatus of the cells after culturing with an anti-synapsin antibody to confirm the induction of the formation of the presynaptic apparatus.

16. The method according to any one of claims 1 to 14, further comprising a step for confirming that the induced synapse containing the presynaptic apparatus has a functionality, after culturing, by any of the following detections of:
(a) detecting a neurotransmitter released from the synapse,
(b) detecting the expression of a protein associated with the release of a neurotransmitter from the synapse, or
(c) adding a labeling agent that labels synaptic vesicles to a medium, and visualizing and detecting the synaptic vesicles that are taken up after the release of a neurotransmitter from the synapse.

17. A microbead in which at least one LRRTM molecule selected from the group consisting of the LRRTM (Leucine-rich repeat transmembrane neuronal protein 2) family molecules or a fusion protein containing the same molecule is fixed to the surface thereof via a linker, the microbead being used for culturing human neurons to induce synapse formation.

18. The microbead according to claim 17, wherein the fusion protein is a fusion protein containing the LRRTM molecule and an Fc region of human IgG and the linker is an anti-human IgG Fc antibody fixed to the surface of the microbead, and the fusion protein containing the LRRTM molecule is fixed to the surface of the microbead via a bond between the Fc region of human IgG and the anti-human IgG Fc antibody.

19. The microbead according to claim 17, wherein the linker is a macromolecule selected from the group consisting of protein, optionally modified polyethylene glycol, optionally modified sugar chain, and optionally modified nucleic acid, and the length of the linker is 10 nm or more.

20. The microbead according to claim 17, wherein the distance between the LRRTM molecule or the LRRTM molecule which is a part of the fusion protein fixed to the surface of the microbead, and the surface of the microbead, is 10 nm or more.

21. The microbead according to any one of claims 17 to 20, wherein the neurons are human peripheral neurons.

22. The microbead according to any one of claims 17 to 20, wherein the neurons are human central neurons.

23. The microbead according to claim 21, wherein the neurons are human motor neurons.

24. The microbead according to claim 22, wherein the neurons are human glutamatergic neurons.

25. The microbead according to any one of claims 17 to 24, wherein the neurons are neurons differentiated from human-derived pluripotent stem cells.

26. The microbead according to claim 25, wherein the human-derived pluripotent stem cells are human iPS cells or human ES cells.

27. A method for screening a drug for a neurological disease, comprising the following steps of:
(i) co-culturing neurons differentiated from iPS cells derived from a patient suffering from the neurological disease together with microbeads in which at least one LRRTM molecule selected from the group consisting of the LRRTM (Leucine-rich repeat transmembrane neuronal protein) family molecules or a fusion protein containing the same molecule is fixed to the surface thereof, to induce the formation of the presynaptic apparatus, wherein the LRRTM molecule or the fusion protein containing the same molecule is fixed to the surface of the microbead via a linker,
(ii) adding a target substance into a medium and culturing it, in the step (i) or after the step (i), and
(iii) then, identifying the effect of the target substance on the formation of the presynaptic apparatus in the neurons.

28. The method according to claim 27, wherein the detection is performed by at least one selected from
(a) observing the morphological state of the presynaptic apparatus formed,
(b) detecting a neurotransmitter released from the presynaptic apparatus
(c) detecting the expression of a protein associated with the release of a neurotransmitter from the presynapse, and
(d) adding a labeling agent that labels synaptic vesicles to a medium, and visualizing and detecting the synaptic vesicles that are taken up after the release of the neurotransmitter from the presynapse.

29. The method according to claim 27 or 28, wherein the fusion protein containing the LRRTM molecule is a fusion protein containing the LRRTM molecule and an Fc region of human IgG and the linker is an anti-human IgG Fc antibody fixed to the surface of the microbead, and the fusion protein containing the LRRTM molecule is fixed to the surface of the microbead via a bond between the Fc region of human IgG and the anti-human IgG Fc antibody.

30. A screening method of performing the method as described in any one of claims 27 to 29 according to high-throughput screening (HTS).

31. A method for screening a substance that promotes or inhibits the release of a neurotransmitter, comprising the following steps of:
(a) co-culturing human neurons together with microbeads in which at least one LRRTM molecule selected from the group consisting of the LRRTM (Leucine-rich repeat transmembrane neuronal protein) family molecules or a fusion protein containing the same molecule is fixed to the surface thereof, to induce the formation of the presynaptic apparatus,
wherein the LRRTM molecule or the fusion protein containing the same molecule is fixed to the surface of the microbead via a linker,
(b) then, culturing the neurons with a target substance added into a medium, and
(c) any one step selected from the group consisting of the following detection steps:
(c-1) detecting a neurotransmitter released into the medium,
(c-2) detecting the expression of a protein associated with the release of a neurotransmitter from the presynapse, and
(c-3) adding a labeling agent that labels synaptic vesicles to a medium, and visualizing and detecting the synaptic vesicles that are taken up after the release of the neurotransmitters from the presynapse.

32. The method according to claim 31, wherein in the step (b), a substance that stimulates the synapse and promotes the neurotransmitter release is further added to a medium.

33. The method according to claim 31 or 32, wherein the fusion protein containing the LRRTM molecule is a fusion protein containing the LRRTM molecule and an Fc region of human IgG and the linker is an anti-human IgG Fc antibody fixed to the surface of the microbead, and the fusion protein containing the LRRTM molecule is fixed to the surface of the microbead via a bond between the Fc region of human IgG and the anti-human IgG Fc antibody.


SEQUENCE LISTING

<110>  Jiksak Bioengineering, Inc.

<120>  Method for inducing synapse formation of nerve cells and
       microbeads used therefor

<130>  JIKS001P

<150>  JP2019-126416
<151>  2019-07-05

<160>  4

<170>  PatentIn version 3.5

<210>  1
<211>  488
<212>  PRT
<213>  Homo sapiens

<400>  1

Ala Pro Ser Gly Cys Pro Gln Leu Cys Arg Cys Glu Gly Arg Leu Leu
1               5                   10                  15

Tyr Cys Glu Ala Leu Asn Leu Thr Glu Ala Pro His Asn Leu Ser Gly
            20                  25                  30

Leu Leu Gly Leu Ser Leu Arg Tyr Asn Ser Leu Ser Glu Leu Arg Ala
        35                  40                  45

Gly Gln Phe Thr Gly Leu Met Gln Leu Thr Trp Leu Tyr Leu Asp His
    50                  55                  60

Asn His Ile Cys Ser Val Gln Gly Asp Ala Phe Gln Lys Leu Arg Arg
65                  70                  75                  80

Val Lys Glu Leu Thr Leu Ser Ser Asn Gln Ile Thr Gln Leu Pro Asn
                85                  90                  95

Thr Thr Phe Arg Pro Met Pro Asn Leu Arg Ser Val Asp Leu Ser Tyr
            100                 105                 110

Asn Lys Leu Gln Ala Leu Ala Pro Asp Leu Phe His Gly Leu Arg Lys
        115                 120                 125

Leu Thr Thr Leu His Met Arg Ala Asn Ala Ile Gln Phe Val Pro Val
    130                 135                 140

Arg Ile Phe Gln Asp Cys Arg Ser Leu Lys Phe Leu Asp Ile Gly Tyr
145                 150                 155                 160

Asn Gln Leu Lys Ser Leu Ala Arg Asn Ser Phe Ala Gly Leu Phe Lys
                165                 170                 175

Leu Thr Glu Leu His Leu Glu His Asn Asp Leu Val Lys Val Asn Phe
            180                 185                 190

Ala His Phe Pro Arg Leu Ile Ser Leu His Ser Leu Cys Leu Arg Arg
        195                 200                 205

Asn Lys Val Ala Ile Val Val Ser Ser Leu Asp Trp Val Trp Asn Leu
    210                 215                 220

Glu Lys Met Asp Leu Ser Gly Asn Glu Ile Glu Tyr Met Glu Pro His
225                 230                 235                 240

Val Phe Glu Thr Val Pro His Leu Gln Ser Leu Gln Leu Asp Ser Asn
                245                 250                 255

Arg Leu Thr Tyr Ile Glu Pro Arg Ile Leu Asn Ser Trp Lys Ser Leu
            260                 265                 270

Thr Ser Ile Thr Leu Ala Gly Asn Leu Trp Asp Cys Gly Arg Asn Val
        275                 280                 285

Cys Ala Leu Ala Ser Trp Leu Asn Asn Phe Gln Gly Arg Tyr Asp Gly
    290                 295                 300

Asn Leu Gln Cys Ala Ser Pro Glu Tyr Ala Gln Gly Glu Asp Val Leu
305                 310                 315                 320

Asp Ala Val Tyr Ala Phe His Leu Cys Glu Asp Gly Ala Glu Pro Thr
                325                 330                 335

Ser Gly His Leu Leu Ser Ala Val Thr Asn Arg Ser Asp Leu Gly Pro
            340                 345                 350

Pro Ala Ser Ser Ala Thr Thr Leu Ala Asp Gly Gly Glu Gly Gln His
        355                 360                 365

Asp Gly Thr Phe Glu Pro Ala Thr Val Ala Leu Pro Gly Gly Glu His
    370                 375                 380

Ala Glu Asn Ala Val Gln Ile His Lys Val Val Thr Gly Thr Met Ala
385                 390                 395                 400

Leu Ile Phe Ser Phe Leu Ile Val Val Leu Val Leu Tyr Val Ser Trp
                405                 410                 415

Lys Cys Phe Pro Ala Ser Leu Arg Gln Leu Arg Gln Cys Phe Val Thr
            420                 425                 430

Gln Arg Arg Lys Gln Lys Gln Lys Gln Thr Met His Gln Met Ala Ala
        435                 440                 445

Met Ser Ala Gln Glu Tyr Tyr Val Asp Tyr Lys Pro Asn His Ile Glu
    450                 455                 460

Gly Ala Leu Val Ile Ile Asn Glu Tyr Gly Ser Cys Thr Cys His Gln
465                 470                 475                 480

Gln Pro Ala Arg Glu Cys Glu Val
                485

<210>  2
<211>  483
<212>  PRT
<213>  Homo sapiens

<400>  2

Cys Pro Pro Lys Cys Arg Cys Glu Lys Leu Leu Phe Tyr Cys Asp Ser
1               5                   10                  15

Gln Gly Phe His Ser Val Pro Asn Ala Thr Asp Lys Gly Ser Leu Gly
            20                  25                  30

Leu Ser Leu Arg His Asn His Ile Thr Glu Leu Glu Arg Asp Gln Phe
        35                  40                  45

Ala Ser Phe Ser Gln Leu Thr Trp Leu His Leu Asp His Asn Gln Ile
    50                  55                  60

Ser Thr Val Lys Glu Asp Ala Phe Gln Gly Leu Tyr Lys Leu Lys Glu
65                  70                  75                  80

Leu Ile Leu Ser Ser Asn Lys Ile Phe Tyr Leu Pro Asn Thr Thr Phe
                85                  90                  95

Thr Gln Leu Ile Asn Leu Gln Asn Leu Asp Leu Ser Phe Asn Gln Leu
            100                 105                 110

Ser Ser Leu His Pro Glu Leu Phe Tyr Gly Leu Arg Lys Leu Gln Thr
        115                 120                 125

Leu His Leu Arg Ser Asn Ser Leu Arg Thr Ile Pro Val Arg Leu Phe
    130                 135                 140

Trp Asp Cys Arg Ser Leu Glu Phe Leu Asp Leu Ser Thr Asn Arg Leu
145                 150                 155                 160

Arg Ser Leu Ala Arg Asn Gly Phe Ala Gly Leu Ile Lys Leu Arg Glu
                165                 170                 175

Leu His Leu Glu His Asn Gln Leu Thr Lys Ile Asn Phe Ala His Phe
            180                 185                 190

Leu Arg Leu Ser Ser Leu His Thr Leu Phe Leu Gln Trp Asn Lys Ile
        195                 200                 205

Ser Asn Leu Thr Cys Gly Met Glu Trp Thr Trp Gly Thr Leu Glu Lys
    210                 215                 220

Leu Asp Leu Thr Gly Asn Glu Ile Lys Ala Ile Asp Leu Thr Val Phe
225                 230                 235                 240

Glu Thr Met Pro Asn Leu Lys Ile Leu Leu Met Asp Asn Asn Lys Leu
                245                 250                 255

Asn Ser Leu Asp Ser Lys Ile Leu Asn Ser Leu Arg Ser Leu Thr Thr
            260                 265                 270

Val Gly Leu Ser Gly Asn Leu Trp Glu Cys Ser Ala Arg Ile Cys Ala
        275                 280                 285

Leu Ala Ser Trp Leu Gly Ser Phe Gln Gly Arg Trp Glu His Ser Ile
    290                 295                 300

Leu Cys His Ser Pro Asp His Thr Gln Gly Glu Asp Ile Leu Asp Ala
305                 310                 315                 320

Val His Gly Phe Gln Leu Cys Trp Asn Leu Ser Thr Thr Val Thr Val
                325                 330                 335

Met Ala Thr Thr Tyr Arg Asp Pro Thr Thr Glu Tyr Thr Lys Arg Ile
            340                 345                 350

Ser Ser Ser Ser Tyr His Val Gly Asp Lys Glu Ile Pro Thr Thr Ala
        355                 360                 365

Gly Ile Ala Val Thr Thr Glu Glu His Phe Pro Glu Pro Asp Asn Ala
    370                 375                 380

Ile Phe Thr Gln Arg Val Ile Thr Gly Thr Met Ala Leu Leu Phe Ser
385                 390                 395                 400

Phe Phe Phe Ile Ile Phe Ile Val Phe Ile Ser Arg Lys Cys Cys Pro
                405                 410                 415

Pro Thr Leu Arg Arg Ile Arg Gln Cys Ser Met Val Gln Asn His Arg
            420                 425                 430

Gln Leu Arg Ser Gln Thr Arg Leu His Met Ser Asn Met Ser Asp Gln
        435                 440                 445

Gly Pro Tyr Asn Glu Tyr Glu Pro Thr His Glu Gly Pro Phe Ile Ile
    450                 455                 460

Ile Asn Gly Tyr Gly Gln Cys Lys Cys Gln Gln Leu Pro Tyr Lys Glu
465                 470                 475                 480

Cys Glu Val

<210>  3
<211>  551
<212>  PRT
<213>  Homo sapiens

<400>  3

Glu Arg Gly Cys Pro Lys Gly Cys Arg Cys Glu Gly Lys Met Val Tyr
1               5                   10                  15

Cys Glu Ser Gln Lys Leu Gln Glu Ile Pro Ser Ser Ile Ser Ala Gly
            20                  25                  30

Cys Leu Gly Leu Ser Leu Arg Tyr Asn Ser Leu Gln Lys Leu Lys Tyr
        35                  40                  45

Asn Gln Phe Lys Gly Leu Asn Gln Leu Thr Trp Leu Tyr Leu Asp His
    50                  55                  60

Asn His Ile Ser Asn Ile Asp Glu Asn Ala Phe Asn Gly Ile Arg Arg
65                  70                  75                  80

Leu Lys Glu Leu Ile Leu Ser Ser Asn Arg Ile Ser Tyr Phe Leu Asn
                85                  90                  95

Asn Thr Phe Arg Pro Val Thr Asn Leu Arg Asn Leu Asp Leu Ser Tyr
            100                 105                 110

Asn Gln Leu His Ser Leu Gly Ser Glu Gln Phe Arg Gly Leu Arg Lys

Leu Leu Ser Leu His Leu Arg Ser Asn Ser Leu Arg Thr Ile Pro Val
    130                 135                 140

Arg Ile Phe Gln Asp Cys Arg Asn Leu Glu Leu Leu Asp Leu Gly Tyr
145                 150                 155                 160

Asn Arg Ile Arg Ser Leu Ala Arg Asn Val Phe Ala Gly Met Ile Arg
                165                 170                 175

Leu Lys Glu Leu His Leu Glu His Asn Gln Phe Ser Lys Leu Asn Leu
            180                 185                 190

Ala Leu Phe Pro Arg Leu Val Ser Leu Gln Asn Leu Tyr Leu Gln Trp
        195                 200                 205

Asn Lys Ile Ser Val Ile Gly Gln Thr Met Ser Trp Thr Trp Ser Ser
    210                 215                 220

Leu Gln Arg Leu Asp Leu Ser Gly Asn Glu Ile Glu Ala Phe Ser Gly
225                 230                 235                 240

Pro Ser Val Phe Gln Cys Val Pro Asn Leu Gln Arg Leu Asn Leu Asp
                245                 250                 255

Ser Asn Lys Leu Thr Phe Ile Gly Gln Glu Ile Leu Asp Ser Trp Ile
            260                 265                 270

Ser Leu Asn Asp Ile Ser Leu Ala Gly Asn Ile Trp Glu Cys Ser Arg
        275                 280                 285

Asn Ile Cys Ser Leu Val Asn Trp Leu Lys Ser Phe Lys Gly Leu Arg
    290                 295                 300

Glu Asn Thr Ile Ile Cys Ala Ser Pro Lys Glu Leu Gln Gly Val Asn
305                 310                 315                 320

Val Ile Asp Ala Val Lys Asn Tyr Ser Ile Cys Gly Lys Ser Thr Thr
                325                 330                 335

Glu Arg Phe Asp Leu Ala Arg Ala Leu Pro Lys Pro Thr Phe Lys Pro
            340                 345                 350

Lys Leu Pro Arg Pro Lys His Glu Ser Lys Pro Pro Leu Pro Pro Thr
        355                 360                 365

Val Gly Ala Thr Glu Pro Gly Pro Glu Thr Asp Ala Asp Ala Glu His

370                 375                 380

Ile Ser Phe His Lys Ile Ile Ala Gly Ser Val Ala Leu Phe Leu Ser
385                 390                 395                 400

Val Leu Val Ile Leu Leu Val Ile Tyr Val Ser Trp Lys Arg Tyr Pro
                405                 410                 415

Ala Ser Met Lys Gln Leu Gln Gln Arg Ser Leu Met Arg Arg His Arg
            420                 425                 430

Lys Lys Lys Arg Gln Ser Leu Lys Gln Met Thr Pro Ser Thr Gln Glu
        435                 440                 445

Phe Tyr Val Asp Tyr Lys Pro Thr Asn Thr Glu Thr Ser Glu Met Leu
    450                 455                 460

Leu Asn Gly Thr Gly Pro Cys Thr Tyr Asn Lys Ser Gly Ser Arg Glu
465                 470                 475                 480

Cys Glu Ile Pro Leu Ser Met Asn Val Ser Thr Phe Leu Ala Tyr Asp
                485                 490                 495

Gln Pro Thr Ile Ser Tyr Cys Gly Val His His Glu Leu Leu Ser His
            500                 505                 510

Lys Ser Phe Glu Thr Asn Ala Gln Glu Asp Thr Met Glu Thr His Leu
        515                 520                 525

Glu Thr Glu Leu Asp Leu Ser Thr Ile Thr Thr Ala Gly Arg Ile Ser
    530                 535                 540

Asp His Lys Gln Gln Leu Ala
545                 550

<210>  4
<211>  560
<212>  PRT
<213>  Homo sapiens

<400>  4

Gln Arg Ala Cys Pro Lys Asn Cys Arg Cys Asp Gly Lys Ile Val Tyr
1               5                   10                  15

Cys Glu Ser His Ala Phe Ala Asp Ile Pro Glu Asn Ile Ser Gly Gly
            20                  25                  30

35                  40                  45

Asn Gln Phe Ala Gly Leu Asn Gln Leu Ile Trp Leu Tyr Leu Asp His
    50                  55                  60

Asn Tyr Ile Ser Ser Val Asp Glu Asp Ala Phe Gln Gly Ile Arg Arg
65                  70                  75                  80

Leu Lys Glu Leu Ile Leu Ser Ser Asn Lys Ile Thr Tyr Leu His Asn
                85                  90                  95

Lys Thr Phe His Pro Val Pro Asn Leu Arg Asn Leu Asp Leu Ser Tyr
            100                 105                 110

Asn Lys Leu Gln Thr Leu Gln Ser Glu Gln Phe Lys Gly Leu Arg Lys
        115                 120                 125

Leu Ile Ile Leu His Leu Arg Ser Asn Ser Leu Lys Thr Val Pro Ile
    130                 135                 140

Arg Val Phe Gln Asp Cys Arg Asn Leu Asp Phe Leu Asp Leu Gly Tyr
145                 150                 155                 160

Asn Arg Leu Arg Ser Leu Ser Arg Asn Ala Phe Ala Gly Leu Leu Lys
                165                 170                 175

Leu Lys Glu Leu His Leu Glu His Asn Gln Phe Ser Lys Ile Asn Phe
            180                 185                 190

Ala His Phe Pro Arg Leu Phe Asn Leu Arg Ser Ile Tyr Leu Gln Trp
        195                 200                 205

Asn Arg Ile Arg Ser Ile Ser Gln Gly Leu Thr Trp Thr Trp Ser Ser
    210                 215                 220

Leu His Asn Leu Asp Leu Ser Gly Asn Asp Ile Gln Gly Ile Glu Pro
225                 230                 235                 240

Gly Thr Phe Lys Cys Leu Pro Asn Leu Gln Lys Leu Asn Leu Asp Ser
                245                 250                 255

Asn Lys Leu Thr Asn Ile Ser Gln Glu Thr Val Asn Ala Trp Ile Ser
            260                 265                 270

Leu Ile Ser Ile Thr Leu Ser Gly Asn Met Trp Glu Cys Ser Arg Ser
        275                 280                 285

Ile Cys Pro Leu Phe Tyr Trp Leu Lys Asn Phe Lys Gly Asn Lys Glu
    290                 295                 300

Ser Thr Met Ile Cys Ala Gly Pro Lys His Ile Gln Gly Glu Lys Val
305                 310                 315                 320

Ser Asp Ala Val Glu Thr Tyr Asn Ile Cys Ser Glu Val Gln Val Val
                325                 330                 335

Asn Thr Glu Arg Ser His Leu Val Pro Gln Thr Pro Gln Lys Pro Leu
            340                 345                 350

Ile Ile Pro Arg Pro Thr Ile Phe Lys Pro Asp Val Thr Gln Ser Thr
        355                 360                 365

Phe Glu Thr Pro Ser Pro Ser Pro Gly Phe Gln Ile Pro Gly Ala Glu
    370                 375                 380

Gln Glu Tyr Glu His Val Ser Phe His Lys Ile Ile Ala Gly Ser Val
385                 390                 395                 400

Ala Leu Phe Leu Ser Val Ala Met Ile Leu Leu Val Ile Tyr Val Ser
                405                 410                 415

Trp Lys Arg Tyr Pro Ala Ser Met Lys Gln Leu Gln Gln His Ser Leu
            420                 425                 430

Met Lys Arg Arg Arg Lys Lys Ala Arg Glu Ser Glu Arg Gln Met Asn
        435                 440                 445

Ser Pro Leu Gln Glu Tyr Tyr Val Asp Tyr Lys Pro Thr Asn Ser Glu
    450                 455                 460

Thr Met Asp Ile Ser Val Asn Gly Ser Gly Pro Cys Thr Tyr Thr Ile
465                 470                 475                 480

Ser Gly Ser Arg Glu Cys Glu Met Pro His His Met Lys Pro Leu Pro
                485                 490                 495

Tyr Tyr Ser Tyr Asp Gln Pro Val Ile Gly Tyr Cys Gln Ala His Gln
            500                 505                 510

Pro Leu His Val Thr Lys Gly Tyr Glu Thr Val Ser Pro Glu Gln Asp
        515                 520                 525

Glu Ser Pro Gly Leu Glu Leu Gly Arg Asp His Ser Phe Ile Ala Thr
    530                 535                 540

Ile Ala Arg Ser Ala Ala Pro Ala Ile Tyr Leu Glu Arg Ile Ala Asn
545                 550                 555                 560